# EUROPEAN PATENT SPECIFICATION

(11) **EP 1 964 552 B1**
(45) Date of publication and mention of the grant of the patent: **26.08.2009**
(21) Application number: 07090036.0
(22) Date of filing: 02.03.2007
(51) Int. Cl.: A61K 31/135, A61K 31/167, A61K 31/192, A61K 31/196, A61K 31/365, A61K 31/485, A61K 31/616, A61K 9/06, A61P 29/00

(54) **Analgesic composition of topically applied nonsteoridal antiinflammatory drugs and opioids**
Analgesische Zusammensetzung von topisch angewendeten, nichtsteroiden und entzündungshemmenden Arzneimitteln und Opioiden
Composition analgésique de médicaments et opioïdes anti-inflammatoires topiques sans stéroïdes

(43) Date of publication of application: 03.09.2008
(73) Proprietor: Flamek Corp OÜ, 11912 Tallinn (EE)
(72) Inventor: Kolesnikov, Yuri, 11912 Tallinn (EE)
(74) Representative: Schubert, Klemens

(56) References cited:
- KOLESNIKOV YURI A ET AL: "The synergistic analgesic interactions between hydrocodone and ibuprofen." ANESTHESIA AND ANALGESIA DEC 2003, vol. 97, no. 6, December 2003 (2003-12), pages 1721-1723, XP002446156 ISSN: 0003-2999
- MASON LORNA ET AL: "Topical NSAIDs for acute pain: a meta-analysis" BMC FAMILY PRACTICE, BIOMED CENTRAL, LONDON, GB, vol. 5, no. 1, 17 May 2004 (2004-05-17), page 1-9, XP021003603 ISSN: 1471-2296
- MASON LORNA ET AL: "Topical NSAIDs for chronic musculoskeletal pain: systematic review and meta-analysis" BMC MUSCULOSKELETAL DISORDERS, BIOMED CENTRAL, LONDON, GB, vol. 5, no. 1, 19 August 2004 (2004-08-19), page 1-8, XP021005598 ISSN: 1471-2474

## Description

### Field of invention

The present invention relates to a topical pharmaceutical composition, formulated with at least one nonsteroidal antiinflammatory drug and at least one opioid analgesic, and to methods of providing pain relief to a subject through topical administration of the composition in an amount and duration sufficient to synergistically potentiate an antinociceptive response.

### Background art

The mechanism by which inflammatory prostaglandins enhance pain perception is incompletely understood. The enhancement of pain perception occurs as the result of prostaglandin-mediated sensitization of the peripheral and central nervous systems. Arachidonic acid is derived from normally sequestered membrane phospholipids. Trauma or inflammation exposes membrane-bound phospholipids to phospholipase A2, which converts them into arachidonic acid. COX-2 converts arachidonic acid into inflammatory prostoglandins (Svensson CI, Yaksh TL. The spinal phospholipase-cyclooxygenase-prostanoid cascade in nociceptive processing. Annu Rev Pharmacol Toxicol.2002; 42:553 -583). Animal models have demonstrated that COX-2 is induced in the dorsal horn of the spinal cord following the peripheral injury (Hefferan MP, Carter P, Haley M, Loomis CW. Spinal nerve injury activates prostaglandin synthesis in the spinal cord that contributes to early maintenance of tactile allodynia. Pain.2003; 101 (1-2) :139 -147.) PGE₂ is a potent vasodilator and hyperalgesic agent. Its vasoactive effects are enhanced by synergistic actions with other inflammatory mediators such as bradykinin and histamine . The hyperalgesia produced by PGE₂ to mechanical stimuli and to other inflammatory mediators may explain the mechanism of postoperative pain (Samad TA, Moore KA, Sapirstein A, Billet S, Allchorne A, Poole S, et al. Interleukin-1beta-mediated induction of Cox-2 in the CNS contributes to inflammatory pain hypersensitivity. Nature.2001; 410:471 -475). Because prostaglandins act via a number of receptors coupled with second messengers, the prostonoid receptors for PGE₂ are probably important for the effect on sensory neurons. This process is known as peripheral sensitization *(*Hefferan MP, Carter P, Haley M, Loomis CW. Spinal nerve injury activates prostaglandin synthesis in the spinal cord that contributes to early maintenance of tactile allodynia. Pain.2003; 101(1-2):139 -147 ). Neuropathic pain arising from lesions to peripheral nerves poses a serious clinical problem. Most analgesics used to treat this syndrome have either a relatively poor therapeutic action or marked side effects, including the tricyclic antidepressants (Fishbain DA. Analgesic effects of antidepressants. J Clin Psychiatry. 2003;64: 96-97)*,* diverse anticonvulsant agents, sodium channel blockers, and opioids ( Hewitt DJ. The use of NMDA-receptor antagonists in the treatment of chronic pain. Clin J Pain . 2000;16(2 suppl):S73-S79*).* Although anecdotal evidence suggests that nonsteroidal anti-inflammatory drugs (NSAIDs), widely used for inflammatory pain, have no efficacy in neuropathic pain, no randomized double blind clinical trials have tested this. Typical examples of the nonsteroidal anti inflammatory propionic acid derivatives include ketoprofen, ibuprofen, diclofenac, flurbiprofen, naproxen, fenoprofen, benoxaprofen, indoprofen. pirprofen, carprofen, oxaprozin, pranoprofen, suprofen, alminoprofen, butibufen and fenbufen, ketorolac, celebrex, rofecoxib, etc. They have potent antiinflammatory and analgesic activities and are widely used for the treatment of rheumatic arthritis and its related conditions. Conventionally, these drugs have been administered orally in the form of solid preparations such as tablets and capsules. However, they have accompanied systemic side effects or gastrointestinal irritation following their oral administration. In order to reduce these side effects, these drugs have been formulated as transdermal preparations based on the fact that the skin permeability of these nonsteroidal antiinflammatory drugs are known to be relatively higher than other nonsteroidal antiinflammatory drugs. For examples, JP 58-39616, JP 58-83622, JP 58-103311, JP 61-238723 and U.S. Pat. Nos. 4,393,076 and 4,534,980 disclosed formulating nonsteroidal antiinflammatory drugs of propionic acid derivatives into transdermal preparations, generally an ointment or a cream. They claimed that the systemic side effects and gastrointestinal irritation of these drugs were significantly reduced while satisfied therapeutic effects were obtained. In these patents, they usually used carboxyvinyl polymer or hydroxypropylmethylcellulose as a gel base in the formulation of transdermal preparations of the drugs. However, the skin permeations of the drugs from their preparations were not enough to achieve pharmacological effects comparable to the oral administration of the drugs due to the low percutaneous absorption of the drugs from the preparations and, thus, a large amount of the preparation needed to be applied to achieve a desired efficacy. Many other chemical and pharmaceutical compositions are known to produce antinociceptive effects. These include, for instance, steroids; non-steroidal anti-inflammatory drugs; local anesthetics; and opioids. These antinociceptive drug classes are useful for modulating many different types of pain, including postoperative, acute, chronic and inflammatory pain. Pain can be alleviated systemically for instance by ingestion or parenteral administration of a suitable composition or, at the site of the pain for instance, by local or topical administration thereof. Opiates are drugs derived from opium and include morphine, codeine and a wide variety of semisynthetic opioid congeners derived from these and from the baine, another component of opium. Opioids include the opiates and all agonists and antagonists with morphine-like activity and naturally occurring endogenous and synthetic opioid peptides (Miyoshi HR, Leckband SG. Systemic opioid analgesics. In: Loeser JD, ed. Bonica's Management of Pain. Philadelphia, PA: Lippincott Williams & Wilkins; 2001: 1682-1709*).* Although morphine and other morphine-like opioid agonists are commonly used to produce analgesia, the severity and high incidence of side effects limits their use. The analgesic effects of morphine are transduced through opioid receptors in the central nervous system (CNS), located at both spinal and multiple supraspinal sites (Pert CB, Snyder SH: Opiate receptor, demonstration in nervous tissue. Science 1973; 179:1101-1104*).* Morphine and its agonists induce profound analgesia when administered intrathecally or instilled locally into the dorsal horn of the spinal cord (Lewis J, Mansour A, Khachaturian H, Watson SJ, Akil H. Opioids and pain regulation. Pain Headache. 1987;9:129-159*).*

Several mechanisms of action are believed to mediate the inhibition of nociceptive reflexes from reaching higher centers of the brain, including the inhibition of neurotransmitter release by opioid receptors on the termini of primary afferent nerves and post-synaptic inhibitory actions on interneurons and on the out-put neurons of the spinothalamic tract. However, opiates can interfere with normal gastrointestinal functioning. Systemic morphine decreases both gastric motility and stomach secretion of hydrochloric acid. Morphine can delay passage of gastric contents through the duodenum for as long as 12 hours. Morphine also decreases biliary, pancreatic, and intestinal secretions and delays the digestion of food in the small intestine. Propulsive peristaltic waves in the colon are diminished or abolished after administration of morphine and commonly, constipation occurs. For a detailed review of the physiologic effects of morphine, see Reisine and Pasternak ,1996 Goodman & Gilnan's, The pharmacological basis of therapeutics, Ninth Edition (Hardman et al. eds.) McGraw-Hillpp.521-555*.*

Systemic morphine also exerts effects on the immune system. The most firmly established immunologic effect of morphine is its ability to inhibit the formation of human lymphocyte rosettes. The administration of morphine to animals causes suppression of the cytotoxic activity of natural killer cells and enhances the growth of implanted tumors. These effects appear to be mediated by actions within the CNS (Sibinga et al. 1988 Annu. Rev. Immunol. 6:219.). Additionally, systemic morphine provokes the release of histamines, which can cause hypotension. Morphine depresses respiration, at least in part by direct effects on brainstem regulatory systems. In humans, death from morphine poisoning is nearly always due to respiratory arrest. Opioid antagonists can produce a dramatic reversal of severe respiratory depression; naloxone is currently the treatment of choice. High doses of morphine and related opioids can produce convulsions that are not always relieved by naloxone (*see* Reisine and Pasternak (1996) Goodman & Gilnan's, The pharmacological basis of therapeutics, Ninth Edition (Hardman et al. eds.)McGraw-Hillpp.521-555*)*.

In addition to morphine, a variety of opioids are suitable for clinical use. These include, but are not limited to, Levorphanol, Meperidine, Fentanyl, Methadone, Codeine, Propoxyphene, Hydrocodone, Hydromorphone, Oxycodone, Oxymorphone, Loperamide, Tramadol and various opioid peptides. Certain opioids are mixed agonists/antagonists and partial agonists. These include pentazocine, nalbuphine, butorphanol, and buprenorphine. Morphine produces analgesia primarily through the mu-opioid receptor. However, among the opioid receptors, there is substantial overlap of function as well as of cellular distribution.

The mu-opioid receptor mediates the actions of morphine and morphine-like opioids, including most clinical analgesics. In addition to morphine, several highly selective agonists have been developed for mu-opioid receptors, including [D-Ala² ,MePhe⁴ ,Gly⁵-ol]enkephalin (DAMGO), levorphanol, etorphine, fentanyl, sufentanil, bremazocine, tramadol and methadone. Mu-opioid receptor antagonists include naloxone, naltrexone, D-Phe-Cys-Try-D-Trp-Orn-Thr-Pen-Thr-NH2 (CTOP), diprenorphine, [beta]-finaltrexamine, naloxonazine, nalorphine, nalbuphine, and naloxone benzoylhydrazone. Differential sensitivity to antagonists, such as naloxonazine, indicates the pharmacologic distinctions between the mu-opioid receptor subtypes, mu, and mu2. Several of the opioid peptides also interact with mu-opioid receptors. Because of extensive investigations with attempts to improve the shortcomings of the existing patents and products, the present inventors have succeeded in establishing novel topical formulations containing a nonsteroidal antiinflammatory drug in conjunction with synergistic doses of opioids and transdermal delivery system. To improve skin penetration of opioids and NSAIDs combination of the propylene glycol and lauric acid has been used. The formulations have high skin permeation rate and, thus, excellent antiinflammatory and analgesic activities comparable to its oral administration.

In the publication or KOLESNIKOV YURI A. ET AL: "The synergistic analgesic interactions between hydrocodone and ibuprofen." ANESTHESIA AND ANALGESIA DEC 2003, vol. 97, no. 6, December 2003 (2003-12), pages 1721-1723, it is disclosed that hydrocodone and ibuprofen administered subcutaneously have an synergistic effect. No topical application is disclosed.

In the publications of MASON LORNA ET AL: "Topical NSAIDs for acute pain: a meta-analysis" BMC FAMILY PRACTICE, BIOMED CENTRAL, LONDON, GB, vol. 5, no. 1, 17 May 2004 (2004-05-17), page 1-9, and "Topical NSAIDs for chronic musculoskeletal pain: systematic review and meta-analysis" BMC MUSCULOSKELETAL DISORDERS, BIOMED CENTRAL, LONDON, GB, vol. 5, no. 1, 19 August 2004 (2004-08-19), page 1-8, it is disclosed that topical NSAIDs were effective and safe in treating acute painful or chronic musculoskeletal conditions for one or two weeks, respectively.

### Disclosure of invention

It is an object of the present invention to provide a pharmaceutical formulation comprising at least two compounds, one effecting opioid analgesia and one effecting cyclooxygenase 1 and 2 activity, in amounts sufficient to potentiate an antinociceptive response when both compounds are topically administered in a physislogically acceptable topical excipient, wherein the opioid is selected from the group consisting of morphine, hydrocodone, and methadone, and wherein the cyclooxygenase 1 and 2 activity effecting compound is a non-steroidal anti-inflammatory drug (NSAID), selected from the group consisting of ibuprofen, and disclofenac.

An especially preferred embodiment of the present invention is **characterized in that** the analgesic is morphine and the NSAID is ibuprofen.

An especially preferred embodiment of the present invention is **characterized in that** the analgesic is morphine and the NSAID is diclofenac.

An especially preferred embodiment of the present invention is **characterized in that** the analgesic is methadone and the NSAID is ibuprofen.

An especially preferred embodiment of the present invention is **characterized in that** the analgesic is methadone and the NSAID is diclofenac.

An especially preferred embodiment of the present invention is **characterized in that** the analgesic is hydrocodone and the NSAID is diclofenac.

An especially preferred embodiment of the present invention is **characterized in that** the analgesic is hydrocodone and the NSAID is ibuprofen.

According to the present invention it is preferred, that the opioid analgesic is present in a dose of about 0.001% to about 10%.

According to the present invention it is preferred, that the NSAIDs is present in a dose of about 0.01% to about 20%.

A preferred topical formulation according to the present invention is in the form of solution, suspension, gel, emugel, cream, ointment, lotion, spray or transdermal patch.

It is also preferred according to the invention that the topical combination of the skin penetration enhancers is preferred, wherein combination of propylene glycol and lauric acid in ratio 10:1 work synergistically.

A further object of the present invention is the use of at least two compounds, one effecting opioid analgesia and one effecting cyclooxygenase 1 and 2 activity, for the production of a topical pharmaceutical combination to prevent or relieve acute incisional pain and chronic peripheral neuropathy in a patient in need of such treatment.

A further object of the present invention is also the use of at least two compounds, one effecting opioid analgesia and one effecting cyclooxygenase 1 and 2 activity, for the production of a topical pharmaceutical combination to prevent or relieve neuropathic inflammation in a patient in need of such treatment.

It is especially preferred to use the pharmaceutical composition, wherein the administration of the pharmaceutical composition is directed to cutaneous, mucosal, vaginal, rectal, ocular, or nasal surfaces.

In other words, the present invention encompasses pharmaceutical compositions comprising at least two compounds, at least one effecting opioid analgesia and at least one effecting on cyclooxygenase 1 and 2 activity, in amounts sufficient to potentiate an antinociceptive response when both compounds are topically administered in a physiologically acceptable topical excipient. The invention further encompasses a method of providing topical analgesia to a subject comprising topical administration of a pharmaceutical composition comprising at least two compounds, at least one effecting opioid analgesia and at least one effecting cyclooxygenase 1 and 2 activity, wherein the pharmaceutical composition is administered in a physiologically acceptable topical excipient and in an amount and a duration sufficient to potentiate an antinociceptive response.

The topical gel formulation of the present invention can be prepared by dissolving a mixture comprising a nonsteroidal antiinflammatory drug of a propionic acid derivative such as ketoprofen, flurbiprofen, ibuprofen, naproxen, fenoprofen, benoxaprofen, indoprofen, pirprofen, carprofen, oxaproztn, pranoprofen, suprofen, alminoprofen, butibufen, diclofenac, ketorolac, aspirin, bextra, celebrex, vioxx and acetominophen in conjunction with opioids such as morphine, methadone, meperidine, tramadol, buprenorphine, pentasozine, hydromorphone, hydrocodone, oxycodone, fentanyl, sufentanyl, loperamide, naloxone and naltrexone. To improve skin penetration of selected synergistic combination of the NSAIDs and opioids we have used poloxamer; and one or more agents selected from lower alcohol, glycerin, propylene glycol and polyethylene glycol; one or more agent enhancers selected from fatty acids, fatty alcohols and menthol.

More specifically, the lower alcohol used in the present invention may be ethanol and isopropyl alcohol, and poloxamer derivatives may be poloxamer 407 and poloxamer 338, poloxamer 237 and others. The concentrated aqueous solution of poloxamer, used as a gel forming agent of this invention, is a low viscous transparent liquid at refrigerator temperature or lower, but turns to a clear semisolid gel on heating to room or body temperature. The polymer also possesses several properties, which make it particularly suitable for use in the formulation of transdermal dosage forms. These include low toxicity and skin irritation, excellent compatibility with other chemicals, high solubilizing capacity for different drugs and good drug release characteristics. Polyethylene glycol may be polyethylene glycol 200, polyethylene glycol 300, polyethylene glycol 400, polyethylene glycol 600, polyethylene glycol 1000 and others. Fatty acids may be lauric acid, oleic acid, captic acid, myristic acid and others, and fatty alcohols may be lauryl alcohol, oleyl alcohol and others.

In the present invention, the pH of the gel may be 4-8 which is usual for the conventional gel forming agent, but it is desirable to use a buffer solution having a pH value around the pKa value of each active compound.

### The best mode for carrying out the invention

Both opioids and NSAID provide peripheral analgesia. Synergistic potentiation of analgesia through topical administration of a topical NSAID/opioid combination offers a new approach to peripheral pain management. Topical administration of a topical NSAID/opioid synergistic drug formulation provides a superior method for the clinical treatment of peripheral pain. It has now been found that topical administration of a composition comprising certain relative amounts of opioids and NSAID results in the synergistic potentiation of peripheral antinociceptive responses. Use of topically administered compositions comprising the proportions of opioids and topical NSAID described and claimed herein provides an important new approach to management of the peripheral pain. The invention encompasses a pharmaceutical composition comprising at least one opioid and at least one NSAID, in amounts sufficient to potentiate an antinociceptive response when the composition is administered topically in a physiologically acceptable topical excipient. As used herein "potentiated antinociceptive response" is a pain-reducing response elicited through the synergistic effect of at least one opioid and at least one NSAID, in which the combined effect is greater than the sum of the effect produced by either agent alone.

The preferred opioid is methadone and, preferably, the opioid in the composition of the present invention is methadone. Other opioids are suitable, including, but not limited to, compounds based on or derived from morphine-like compounds and analogs. The opioid can be, but is not limited to, morphine, heroin, ethylmorphine, hydromorphone, oxymorphone, codeine, hydrocodone, levorphanol, oxycodone, pentazocine, loperamide, propoxyphene, fentanyl, sufentanil, lofentanil, morphine-6-glucuronide, buprenorphine, etorphine, [D-Ala² ,MePhe⁴ ,Gly⁵-ol]enkephalin (DAMGO), butorphanol, nalorphine, nalbuphine, naloxone benzoylhydrazone, naloxone, naltrexone, meperidine, bremazocine, ethylketocyclazocine, U-50488, U-69593, spiradoline, naltrindole, [D-Pen² ,D-Pen⁵] enkephalin (DPDPE), [D-Ala² , Glu⁴] deltorphin, and [D-Ser² ,Leu⁵]enkephalin-Thr⁶ (DSLET), [D-Ala² ,MePhe⁴ ,Gly⁵-ol] enkephalin, and β-endorphin, dynorphin A, dynorphin B and α-neoendorphin and small molecule and combinatorial chemistry products thereof. Ibuprofen is the preferred NSAIDs in the composition of the present invention. The NSAIDs can be any known in the art, including, but not limited to ketoprofen, flurbiprofen, naproxen, fenoprofen, benoxaprofen, indoprofen, pirprofen, carprofen, oxaproztn, pranoprofen, suprofen, alminoprofen, butibufen, diclofenac, ketorolac, acetaminophen, aspirin, celebrex and rofecoxib.

The topical formulations used in the present invention are particularly suitable for formulations as topical preparations. Formulations suitable for topical administration include liquid or semi-liquid preparations suitable for penetration through the skin to the site of where treatment is required. Examples of liquid and semi-liquid preparations include, but are not limited to, topical solutions, liniments, lotions, creams, ointment or paste or gel. Other topical ingredients used in the topical formulation are in general those commonly used and generally recognized by person skilled in the art of topical formulation. Topical solution of the present invention may contain aqueous or oily solution or suspensions. They may be prepared by dissolving the pharmaceutical compound in a suitable aqueous solution, which may also contain a bactericidal agent, a fungicidal agent, or any other suitable preservative, and may preferably include a surface active agent. Suitable solvents for the preparation of an oily solution include glycerol, diluted alcohol, and propylene glycol. Optionally, L-menthol may be added to the topical solution. Lotions and liniments include those suitable for application to the skin containing a sterile aqueous solution and optionally, a bactericide. They may also include an agent to hasten drying and cooling of the solution on the skin, such as alcohol or acetone. They may further include a moisturizer, such as glycerol, or an oil, such as castor oil or arachis oil.

Cream, ointments, or pastes, are semi-solid formulations made by mixing the pharmaceutical with a greasy or non-greasy base. The topical formulation is in finely-divided or powdered form and may be alone or in a aqueous or nonaqueous solution or suspension. The topical formulation may be mixed with the greasy or non-greasy base with the aid of suitable machinery. The base may contain hydrocarbons. Examples of the hydrocarbons include, but are not limited to, hard, soft, or liquid paraffin, glycerol, beeswax, a metallic soap, a mucilage, an oil of natural origin (such as almond, corn, arachis, castor or olive oil), wool fat or its derivative, a fatty acid (such as stearic acid or oleic acid), or a combination thereof. The formulation may also contain a surface active agent, such as an anionic, cationic or non-ionic surfactant. Examples of the surfactants include, but are not limited to, sorbitan esters or polyoxyethylene derivatives thereof (such as polyoxyethylene fatty acid esters) and carboxypolymethylene derivatives thereof (such as carbopol). Suspending agents such as natural gums, cellulose derivatives inorganic materials such as silicaceous silicas, and other ingredients such as lanolin, may also be included. For ointment, polyethylene glycol 540, polyethylene glycol 3350, and propylene glycol may also be used to mixed with the topical formulation.

A gel or emugel formulation includes any gel forming agent commonly used in the pharmaceutical gel formulations. Examples of gel forming agents are cellulose derivatives such as methyl cellulose, hydroxyethyl cellulose, and carboxymethyl cellulose; vinyl polymers such as polyvinyl alcohols, polyvinyl pyrrolidones; carboxypolymethylene derivatives such as carbopol. Further gelling agents that can be used for the present invention are pectins and gums (such as gum arabic and tragacanth, alginates, carrageenates, agar and gelatin). The preferred gelling agent is carbopol. Furthermore, the gel or emugel formulation may contain auxiliary agents commonly used in the kind of formulations such as preservatives, antioxidants, stabilizers, colorants, and perfumes. The analgesic topical formulations of the present invention prepared as above exhibited excellent pharmacological activities as shown in the Experiments below.

The following examples describe the present invention in more detail. The examples do not intend to limit the scope of the invention.

All in vivo studies were carried out in accordance with the Declaration of Helsinki and with the Guide for Care and Use of Laboratory Animals, as adopted and promulgated by the Estonian Põllumajandusministeerium. Male Crl:CD-1® (ICR) BR mice (20-25 g) were purchased from Charles River Laboratories (Germany) and were housed in a 12:12-h light-dark cycle temperature-controlled room with food and water freely available. Drugs were obtained from Sigma (St. Louis, MO, USA). Drugs were applied topically and analgesia assessed as previously described (Kolesnikov et al (2000) J of Pharmacol and Experimental Ther. 295(2): 546-551). Briefly, the distal portion of the tail (2-3cm) was immersed in a propylene glycol/dH₂O(10:1 ratio) alone or propylene glycol and lauric acid combination (10:1) solution containing the indicated drugs for the stated time, usually 2 min. ). Analgesia was defined quantitatively as tail-flick latency for an individual animal that was twice its baseline latency or greater using tail flick apparatus (Ugo Basile, Italy). Baseline latencies typically ranged from 2.5 to 3.5 s, with a maximum cutoff latency of 10 s to minimize tissue damage in analgesic animals. Since analgesia was assessed quantitatively, groups comparisons were performed with the Fisher's exact test (Kolesnikov et al. (1993) Proc. Natl. Acad. Sci. USA 90:5162). ED₅₀ values were determined with GraphPad Software.

### Short description of the Figures

### Fig 1:

### Topical morphine and lidocaine effects in radiant heat tail-flick assay

Groups of mice (*n* ≥ 10) were exposed to the indicated concentration of the free base of lidocaine (1.1 - 8.6 mmol) or morphine (1.5- 15 mmol) for 2 min and tested immediately afterward.

### Fig 2:

### Addition lauric acid to propylene glycol shifts topical lidocain and morphine dose response curve to left

A. Groups of mice (*n* ≥ 10) were exposed to the indicated concentration of the free base of lidocaine dissolved in PG alone (1.1- 8.6 mmol) or in combination of PG/LA (0.2- 2.1 mmol) for 2 min and tested immediately afterward.
B. Other groups of mice (n≥ 10) were exposed to morphine sulphate dissolved in propylene glycol (1.5 - 11.3 mmol) or in combination of propylene glycol and lauric acid (0.15 - 1.5 mmol) for 2 min and tested immediately afterwards.

### Fig 3:

### Time dependence of topical lidocaine and morphine analgesia

A. Groups of mice (*n* ≥ 10) were exposed for 2 min to a fixed concentration of topical lidocaine (0.9 mmol) dissolved either in PG or PG/LA and then were tested in the tail-flick assay immediately after termination of drug exposure.
B. Groups of mice (*n* ≥ 10) were treated for 2 min with fixed dose of morphine (1.5 mmol) dissolved either in PG or PG/LA and then tested in tail-flick assay at the indicated time over 90 min.

### Fig 4:

### Addition lauric acid to propylene glycol shifts topical methadone dose response curve to left and increase the duration of analgesic effect

A. Groups of mice (*n* ≥ 10) were exposed to the indicated concentration of the methadone (0.7 - 14 mmol) dissolved either in PG alone or in PG/LA for 2 min and tested immediately afterward.
B. Other groups of mice (*n* ≥ 10 ) were treated with fixed dose of methadone ( 3.0 mmol) dissolved either in PG or PG/LA and then tested in tail-flick assay at indicated time over 60 min.

### Fig 5:

### Addition lauric acid to propylene glycol shifts topical S-ibuprofen and diclofenac dose response curve to left

A. Groups of mice (*n* ≥ 10) were exposed to the indicated concentration of diclofenac dissolved in PG alone (1.8 - 7.0 mmol) or in combination of PG/LA (0.9 - 3.5 mmol) for 2 min and tested immediately afterward.
B. Other groups of mice (n≥ 10) were exposed to S-ibuprofen dissolved in PG (4.8- 19.2 mmol) or in combination of PG and LA(1.2- 4.8 mmol) for 2 min and tested immediately afterwards.

### Fig 6:

### Time dependence of topical S-ibuprofen and diclofenac analgesia

A. Groups of mice (*n* ≥ 10) were exposed for 2 min to a fixed concentration of topical S-ibuprofen (4.8 mmol) dissolved either in PG or PG/LA and then were tested in the tail-flick assay at the indicated time over 90 min.
B. Groups of mice (*n* ≥ 10) were treated for 2 min with fixed dose of diclofenac (3.5 mmol) dissolved either in PG or PG/LA and then tested in tail-flick assay at the indicated time over 90 min.

### Fig 7:

### Topical diclofenac and morphine interactions

A. Groups of mice received either topical morphine (0.15 mmol; *n* = 10) or diclofenac (0.9 mmol; *n* = 10) alone or both together (*n* = 10) for 2 min. The combination was significantly (*P* < 0.05) more active at peak effect than the sum of two individual agents.
B. Groups of mice (n=30) received either topical morphine (0.15 mmol) or diclofenac (0.9 mmol) or the combination of the two for 2 min and were tested in the tail-flick assay over 90 min.

### Fig 8:

### Topical S-ibuprofen and morphine interactions

A. Groups of mice received either topical morphine (0.15 mmol; *n* = 10) or S-ibuprofen (1.2 mmol; *n* = 10) alone or both together (*n* = 10) for 2 min.
B. Groups of mice (n=30) received either topical morphine (0.15 mmol) or S-ibuprofen (1.2 mmol) or the combination of the two for 2 min and were tested in the tail-flick assay over 90 min.

### Fig 9:

### Topical methadone and diclofenac interactions

A. Groups of mice received either topical methadone (0.7 mmol; *n* = 10) or diclofenac (0.9 mmol; *n* = 10) alone or both together (*n* = 10) for 2 min. The combination was significantly (*P* < 0.01) more active at peak effect than the sum of two individual agents.
B. Groups of mice (n=30) received either topical methadone (0.7 mmol, n=10) or diclofenac (0.9 mmol, n=10) or the combination (n=10) of the two for 2 min and were tested in the tail-flick assay over 90 min.

### Fig 10:

### Topical methadone and S-ibuprofen interactions

A, groups of mice received either topical methadone (0.7 mmol; *n* = 10) or S-ibuprofen (1.2 mmol; *n* = 10) alone or both together (*n* = 10) for 2 min. The combination was significantly (*P* < 0.01) more active at peak effect than the sum of two individual agents.
B. Groups of mice (n=30) received either topical methadone (0.7 mmol, n=10) or S-ibuprofen (1.2 mmol, n=10) or the combination (n=10) of the two for 2 min and were tested in the tail-flick assay over 120 min.

### Fig 11:

### Topical NSAIDs and hydrocodone interactions

A. Groups of mice (n= 20) received topical hydrocodone (0.6-4.4 mmol) dissolved either PG or PG/LA solution for 2 min and then tested immediately afterwards.
B. Groups of mice (n=30) received either topical hydrocodone ( 0.6 mmol, n=10 ) or S-ibuprofen (1.2 mmol, n=10) alone or both together (n=10) for 2 min.
C. Other groups on animals received topical either hydrocodone (0.6 mmol, n=10) or diclofenac ( 0.9 mmol, n=10) or together (n=10) and were tested in the tail-flick assay.

### Addition of lauric acid to propylene glycol improves drug penetration through skin and increase the potency of the lidocaine and opioids and NSAIDs

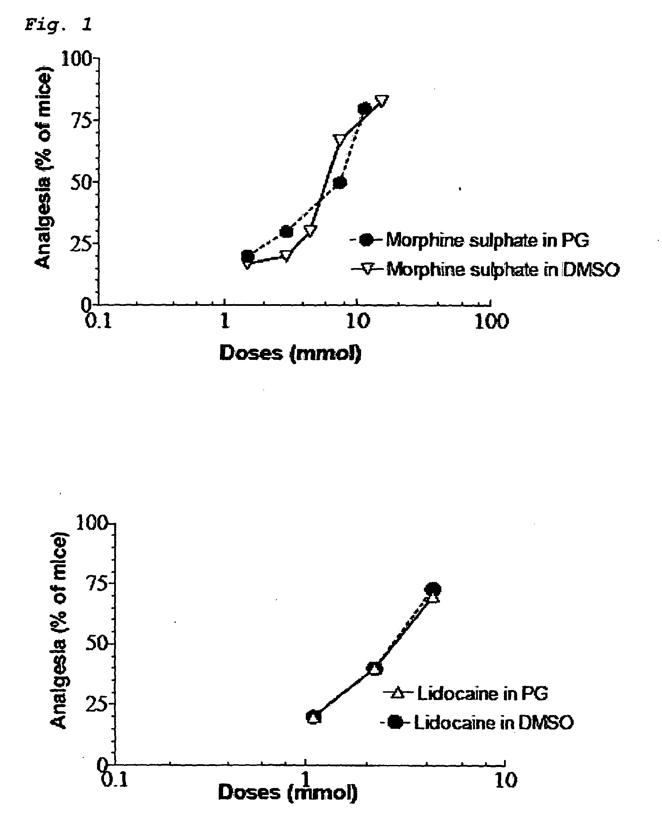

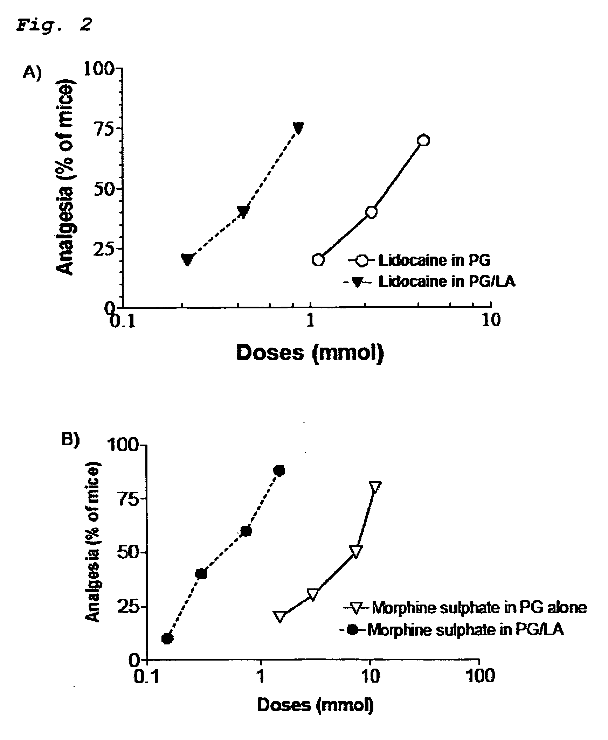

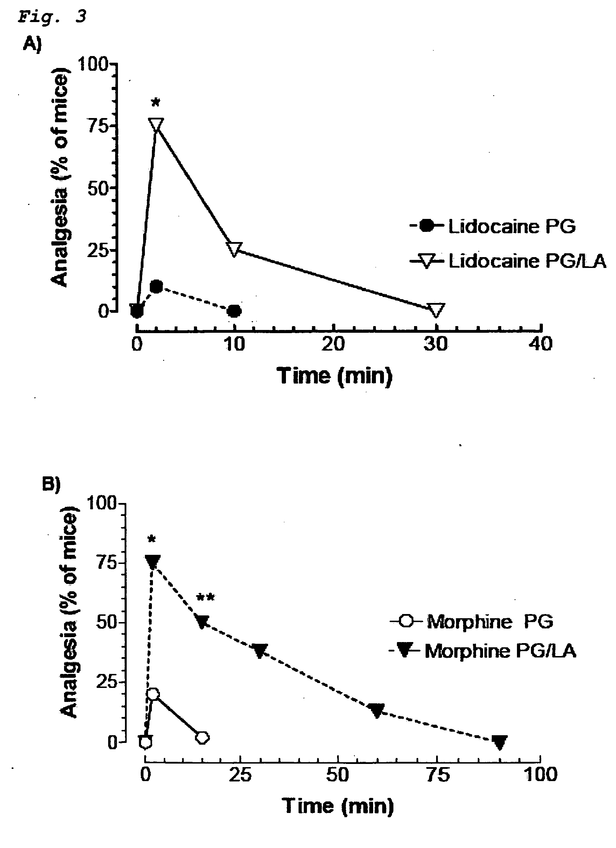

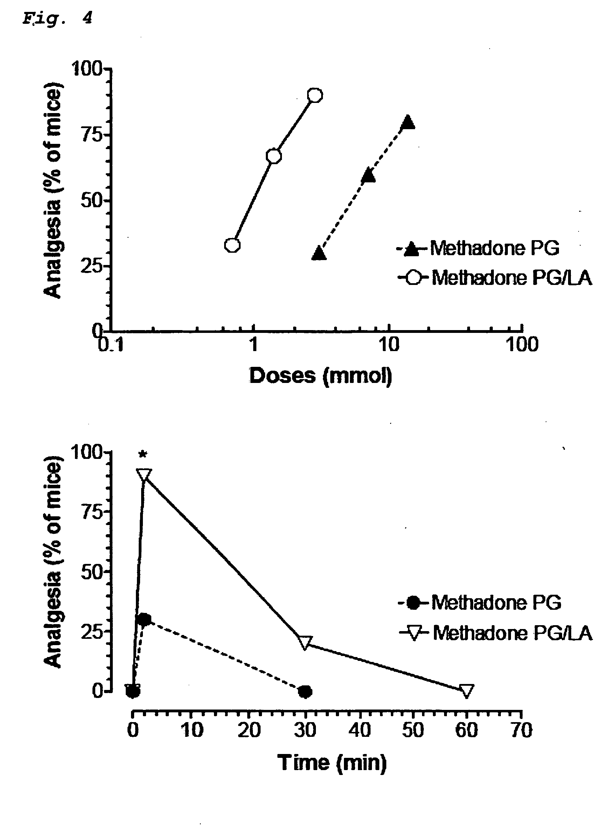

Propylene glycol is a widely used trans-dermal vehicle for number of the drugs. In early studies, we demonstrated the topical lidocaine and morphine analgesia using DMSO as solvent and skin penetration enhancer . DMSO alone was ineffective in our pain model. In current studies, we also found that propylene glycol alone did not increase significantly base line latencies in mice in radiant heat tail-flick assay (data not shown). To ensure local action, in all studies, we examined a region of the tail that was immersed in vehicle and a more proximal segment that was not exposed. Tail-flick latencies from the unexposed portion of the tail were similar to baseline latencies. Testing regions of the tail that were exposed and not exposed to vehicle revealed no significant antinociceptive effect in either location. In comparative studies of potency topically applied drugs using different vehicles, we found no differences in ED₅₀ of the morphine and lidocaine (Fig 1). However, addition of lauric acid to propylene glycol dramatically improves penetration of the morphine and lidocaine, measured as drug's ED₅₀. We observed significant dose response shift to left for lidocaine's ED₅₀ (5-fold shift, Fig 2A, Table 1) and for morphine's ED₅₀ (19-fold shift, Fig 2B, Table 1). In time course studies the distinctions in the potency of the drugs have been even more expressed (Fig 3). Low lidocaine dose in propylene glycol and lauric acid solution produced significant and long acting analgesic effects compared with propylene glycol alone (Fig 3A). Duration of the analgesic activity of the morphine in propylene glycol and lauric acid was far beyond compared with the same dose of the drug dissolved in propylene glycol only (Fig 3B). Methadone produced also analgesic activity in this experimental paradigm. As anticipated, methadone elicited dose dependent (Fig 4A) and naloxone sensitive (data not shown) analgesia after topical administration. Addition of lauric acid to propylene glycol modulated the methadone potency and its duration effect (Fig 4B, Table 1). We observed significant dose response shift to the left (6.7 fold, Table 1). The hydrocodone demonstrated the most potent analgesic activity in this paradigm (Table 1).

**Table 1**

| Addition of the lauric acid to propylene glycol enhances the potency of topical drugs in radiant heat tail flick assay | | | | |
|---|---|---|---|---|
| Analgesics | ED 50 value (95 % CL) | | | |
| | Propylene glycol alone | Propylene glycol and lauric acid | Shift | P value |
| Lidocaine | 2.2 | 0.35 | 6.2 | <0.0001 |
| | (1.6-3.2) mmol | (0.2-0.42)mmol | | |
| | | | | |
| Morphine | 7.6 | 0.4 | 19 | <0.00001 |
| | (4.2- 10.9)mmol | (0.33-0.54)mmol | | |
| | | | | |
| Methadone | 6.7 | 1.0 | 6.7 | <0.001 |
| | (4.8- 8.8) mmol | (0.8-1.2) mmol | | |
| | | | | |
| Hydrocodone | 1.6 | 1.2 | 1.3 | >0.05 |
| | (1.1 - 2.2) mmol | ( 0.8 -1.8) mmol | | |
| | | | | |
| S-ibuprofen | 9.3 | 2.1 | 4.4 | < 0.001 |
| | (6.1- 16.5) mmol | (0.88- 3.3) mmol | | |
| | | | | |
| Diclofenac | 3.3 | 1.1 | 3 | < 0.02 |
| | (1.7-5.3) mmol | ( 0.6-1.78) mmol | | |

### Topical S - ibuprofen and diclofenac analgesia in radiant tail-flick assay

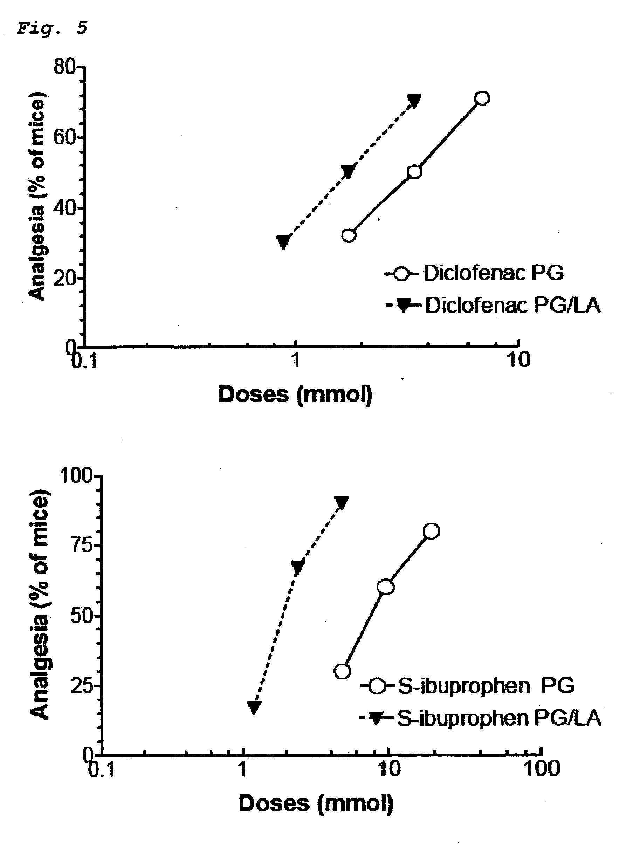

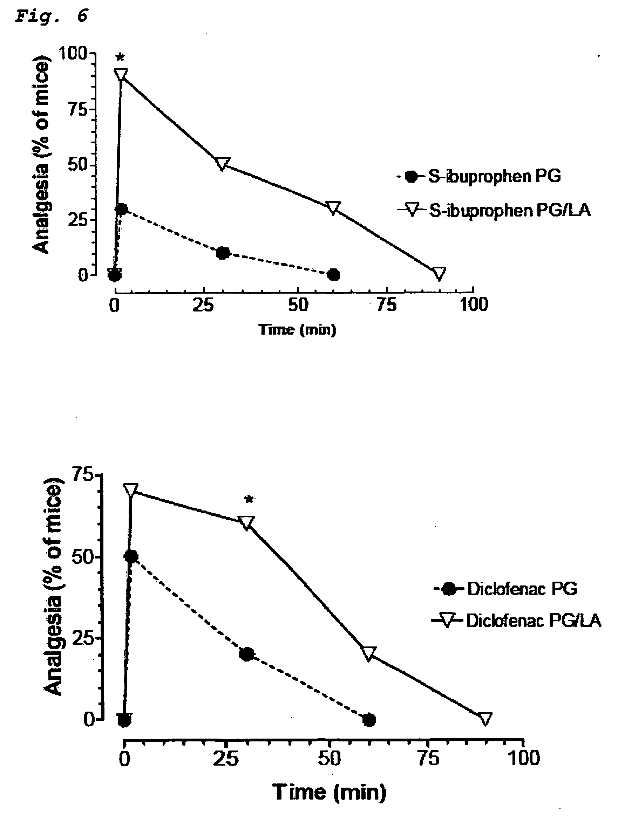

Both S-ibuprofen and diclofenac produced dose dependent analgesia in this paradigm (Fig 5) with ED₅₀ 9.3 and 3.3 mmol, respectively. As anticipated, addition of lauric acid to propylene glycol resulted in to significant increase the potency of the drugs (Table 1). The maximal analgesic effect of S-ibuprofen and diclofenac observed on 2 min after topical application and duration of analgesia was 90 min.

### Topical opioid and S-ibuprofen and diclofenac synergistic interactions in radiant tail flick assay

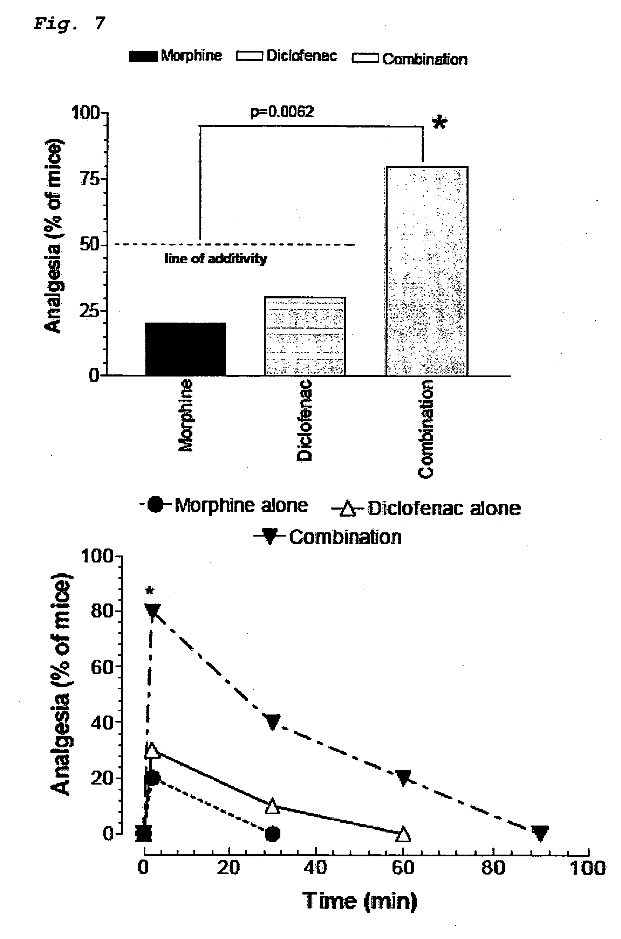

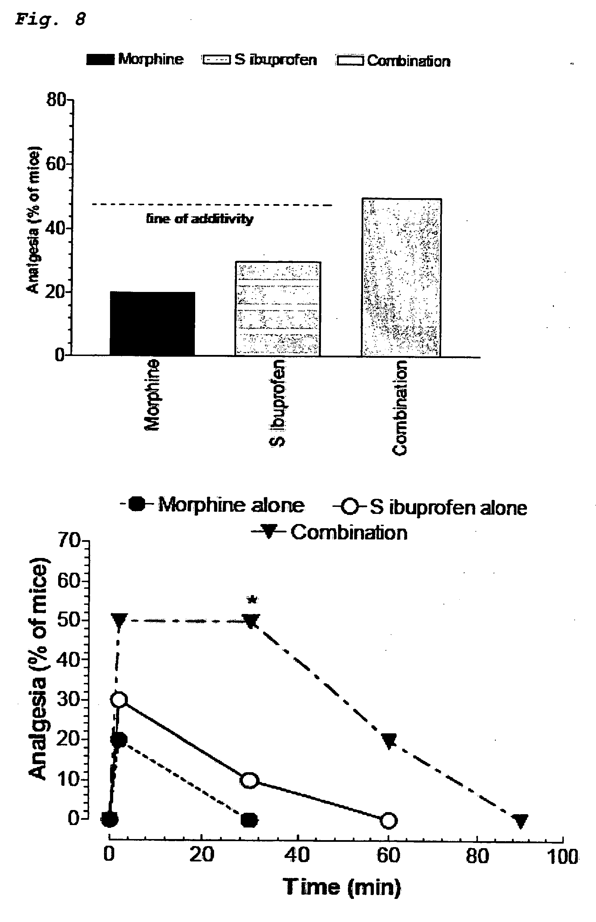

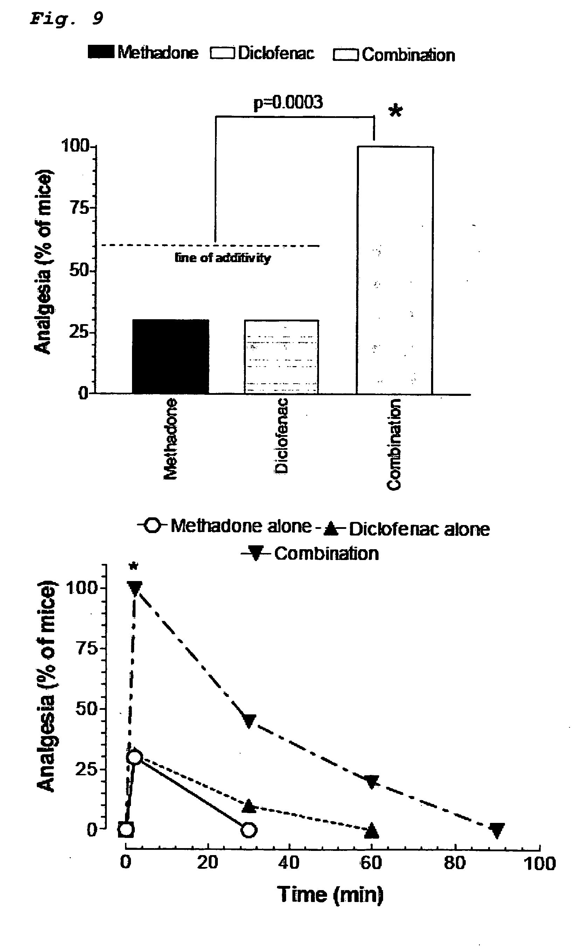

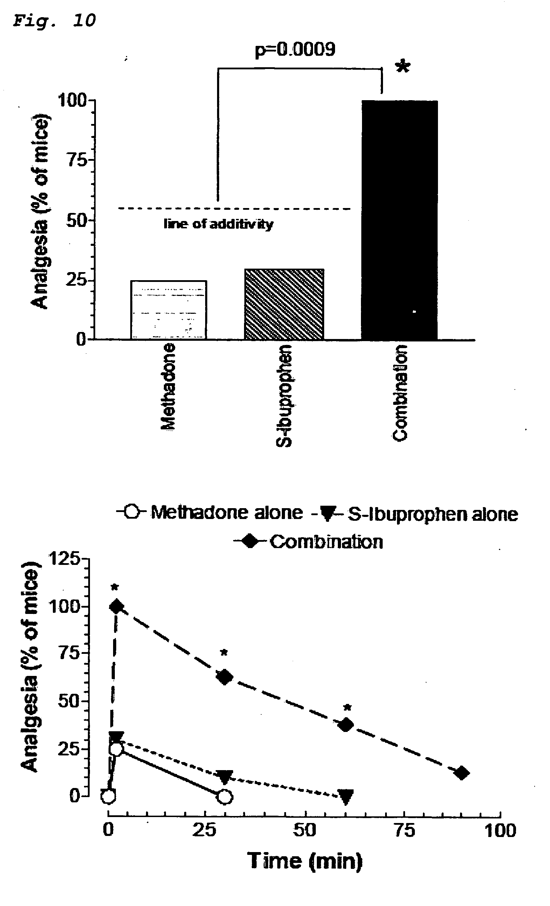

We next assessed potential interactions between opioids and ibuprofen and diclofenac using a fixed, low dose of each. Alone, morphine and diclofenac produced analgesia in 20% and 30% of mice, respectively. However, when the same doses were administered together, the combination produced analgesia in 80% of mice, significantly greater than anticipated from simple additive interactions (50% *versus* 80 %, *p*<0.005) (Fig 7A). Duration of analgesic activity of the combination have also been more pronounced as compared with each drug alone and observed during 90 minutes (Fig 7B). Interesting, morphine and S-ibuprofen combination was additive at 2 min after topical drug administration (50% *versus* 50%), and then became synergistic at 30 min (50% *versus* 10%, p< 0.01) (Fig 8). We next examined possible interactions between methadone and diclofenac and methadone and S-ibuprofen. Alone topical methadone and diclofenac produced analgesia in 30% of mice (Fig 9A). The combination produced analgesia in 100% of mice (100% *versus* 60%, p< 0.001) and analgesic affect observed during 90 min (Fig 9B). Alone topical methadone and S-ibuprofen produced analgesia in 25% and 30 % mice, respectively. The combination elicited analgesia in 100 % of mice (100% *versus* 55%, p<0.001) (Fig 10A) and this analgesic effect lasted during 120 min (Fig 10B).

### Topical hydrocodone potentates S-ibuprofen and diclofenac analgesia in radiant tail flick assay

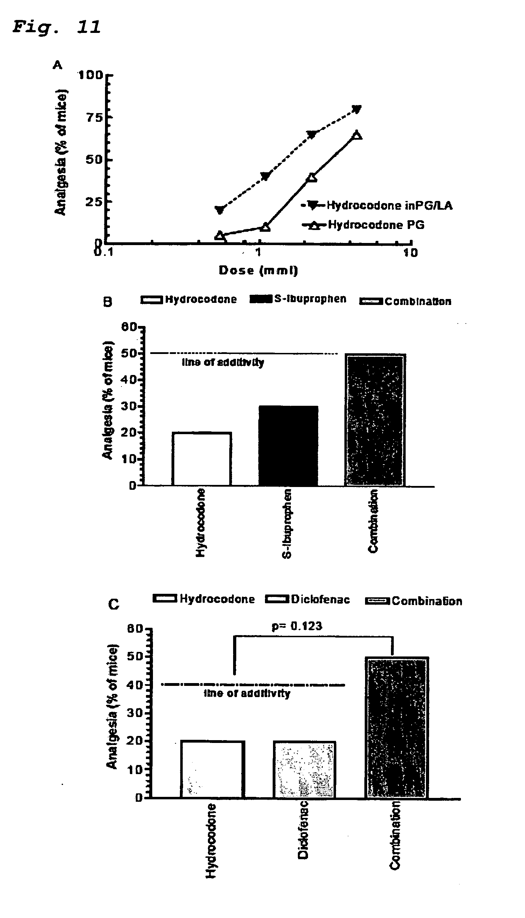

Alone, hydrocodone produced dose dependent response in this paradigm (Fig 11A). Then we assessed potential interactions between hydrocodone and ibuprofen and diclofenac. Alone, hydrocodone and S-ibuprofen produced 20% and 30% of analgesia in mice and combination of the drugs were effective in 50 % of mice, respectively (Fig 11 B), indicating additive interaction in this pain model (50% versus 50%). The combination of hydrocodone and diclofenac produced analgesia in 50% mice, however, it have not reach statistical significance compared with anticipated additive interactions (50% *versus* 40%, p<0.05, Fig 11 C).

## Claims

1. A pharmaceutical formulation comprising at least two compounds, one effecting opioid analgesia and one effecting cyclooxygenase 1 and 2 activity, in amounts sufficient to potentiate an antinociceptive response when both compounds are topically administered in a physiologically acceptable topical excipient, wherein the opioid is selected from the group consisting of morphine, hydrocodone, and methadone, and wherein the cyclooxygenase 1 and 2 activity effecting compound is a non-steroidal anti-inflammatory drug (NSAID), selected from the group consisting of ibuprofen, and diclofenac.

2. The topical pharmaceutical composition according to claim 1, wherein the analgesic is morphine and the NSAID is ibuprofen.

3. The topical pharmaceutical composition according to claim 1, wherein the analgesic is morphine and the NSAID is diclofenac.

4. The topical pharmaceutical composition according to claim 1, wherein the analgesic is methadone and the NSAID is ibuprofen.

5. The topical pharmaceutical composition according to claim 1, wherein the analgesic is methadone and the NSAID is diclofenac.

6. The topical pharmaceutical composition according to claim 1, wherein the analgesic is hydrocodone and the NSAID is diclofenac.

7. The topical pharmaceutical composition according to claim 1, wherein the analgesic is hydrocodone and the NSAID is ibuprofen.

8. The topical pharmaceutical composition according to any of the preceding claims 1 to 7, wherein the opioid analgesic is present in a dose of about 0.001% to about 10%.

9. The topical pharmaceutical composition according to any of the preceding claims 1 to 7, wherein the NSAIDs is present in a dose of about 0.01% to about 20%.

10. The topical formulation according to claim 1, wherein said topical formulation is in the form of solution, suspension, gel, emugel, cream, ointment, lotion, spray or transdermal patch.

11. The topical combination of the skin penetration enhancers according to any of the preceding claims 1 to 10, wherein combination of propylene glycol and lauric acid in ratio 10:1 work synergistically.

12. Use of a pharmaceutical formulation according to claim 1, for the production of a topical pharmaceutical combination to prevent or relieve acute incisional pain and chronic peripheral neuropathy in a patient in need of such treatment.

13. Use of a pharmaceutical formulation according to claim 1, for the production of a topical pharmaceutical combination to prevent or relieve neuropathic inflammation in a patient in need of such treatment.

14. Use according to claim 12 or 13, wherein the administration of the pharmaceutical composition is directed to cutaneous, mucosal, vaginal, rectal, ocular, or nasal surfaces.

## Patentansprüche

1. Pharmazeutische Zubereitung, umfassend wenigstens zwei Verbindungen, von denen eine Opioid-Analgesie und eine Cyclooxigenase-1 und -2-Aktivität bewirkt, in Mengen, die ausreichend sind eine anti-nozizeptive Antwort zu potenzieren, wenn beide Verbindungen in einem physiologisch verträglichen, topischen Hilfsstoff topisch verabreicht werden, worin das Opioid ausgewählt ist aus der Gruppe, bestehend aus Morphin, Hydrokodon und Methadon, und worin die die Cyclooxygenase-1- und -2-Aktivität bewirkende Verbindung ein nicht-steroidales entzündungshemmendes Arzneimittel (NSAID) ist, ausgewählt aus der Gruppe, bestehend aus Ibuprofen und Diclofenac.

2. Topische pharmazeutische Zusammensetzung gemäß Anspruch 1, worin das Analgetikum Morphin ist und das NSAID Ibuprofen ist.

3. Topische pharmazeutische Zusammensetzung gemäß Anspruch 1, worin das Analgetikum Morphin ist und das NSAID Diclofenac ist.

4. Topische pharmazeutische Zusammensetzung gemäß Anspruch 1, worin das Analgetikum Methadon ist und das NSAID Ibuprofen ist.

5. Topische pharmazeutische Zusammensetzung gemäß Anspruch 1, worin das Analgetikum Methadon ist und das NSAID Diclofenac ist.

6. Topische pharmazeutische Zusammensetzung gemäß Anspruch 1, worin das Analgetikum Hydrokodon ist und das NSAID Diclofenac ist.

7. Topische pharmazeutische Zusammensetzung gemäß Anspruch 1, worin das Analgetikum Hydrokodon ist und das NSAID Ibuprofen ist.

8. Topische pharmazeutische Zusammensetzung gemäß irgendeinem der vorangehenden Ansprüche 1 bis 7, worin das Opioid-Analgetikum in einer Dosis von ungefähr 0,001 % bis ungefähr 10 % vorliegt.

9. Topische pharmazeutische Zusammensetzung gemäß irgendeinem der vorangehenden Ansprüche 1 bis 7, worin das NSAID in einer Dosis von ungefähr 0,01 % bis ungefähr 20 % vorliegt.

10. Topische Zubereitung gemäß Anspruch 1, worin die genannte topische Zubereitung in Form einer Lösung, einer Suspension, eines Gels, eines Emugels, einer Creme, einer Salbe, einer Lotion, eines Spray oder eines transdermalen Pflasters vorliegt.

11. Topische Kombination des Hautpenetrationsverstärkers gemäß irgendeinem der Ansprüche 1 bis 10, worin eine Kombination von Propylenglycol und Laurinsäure im Verhältnis von 10:1 synergistisch arbeitet.

12. Verwendung einer pharmazeutischen Zubereitung gemäß Anspruch 1, für die Herstellung einer topischen pharmazeutischen Kombination, zur Linderung oder Prävention akuten schneidenden Schmerzes und chronischer peripherer Neuropathie bei einem Patienten, der einer solchen Behandlung bedarf.

13. Verwendung einer pharmazeutischen Zubereitung gemäß Anspruch 1, für die Herstellung einer topischen pharmazeutischen Kombination, zur Verhinderung oder Linderung neuropathischer Entzündung bei einem Patienten, der einer solchen Behandlung bedarf.

14. Verwendung gemäß Anspruch 12 oder 13, worin die Verabreichung der pharmazeutischen Zusammensetzung auf kutane, mukosale, vaginale, rektale, okulare oder nasale Oberflächen ausgerichtet ist.

## Revendications

1. Formulation pharmaceutique comprenant au moins deux composés, l'un déployant une activité d'analgésique opioïde et l'autre déployant une activité de cyclo-oxygénase 1 et 2, dans des quantités suffisantes pour potentialiser une réponse antinociceptive lorsque les deux composés sont administrés par voie locale dans un excipient local physiologiquement acceptable, l'opioïde étant choisi parmi le groupe constitué par la morphine, l'hydrocodone et la méthadone, et dans laquelle le composé déployant une activité de cyclo-oxygénase 1 et 2 est un médicament anti-inflammatoire non stéroïdien (NSAID) choisi parmi le groupe constitué par l'ibuprofène et le diclofenac.

2. Composition pharmaceutique locale selon la revendication 1, dans laquelle l'analgésique est la morphine et le NSAID est l'ibuprofène.

3. Composition pharmaceutique locale selon la revendication 1, dans laquelle l'analgésique est la morphine et le NSAID est le diclofenac.

4. Composition pharmaceutique locale selon la revendication 1, dans laquelle l'analgésique est la méthadone et le NSAID est l'ibuprofène.

5. Composition pharmaceutique locale selon la revendication 1, dans laquelle l'analgésique est la méthadone et le NSAID est le diclofenac.

6. Composition pharmaceutique locale selon la revendication 1, dans laquelle l'analgésique est l'hydrocodone et le NSAID est le diclofenac.

7. Composition pharmaceutique locale selon la revendication 1, dans laquelle l'analgésique est l'hydrocodone et le NSAID est l'ibuprofène.

8. Composition pharmaceutique locale selon l'une quelconque des revendications précédentes 1 à 7, dans laquelle l'analgésique opioïde est présent en une dose d'environ 0,001 % à environ 10 %.

9. Composition pharmaceutique locale selon l'une quelconque des revendications précédentes 1 à 7, dans laquelle le NSAID est présent en une dose d'environ 0,01 % à environ 20 %.

10. Composition pharmaceutique locale selon la revendication 1, dans laquelle ladite formulation locale est présente sous la forme d'une solution, d'une suspension, d'un gel, d'un émulgel, d'une crème, d'un onguent, d'une lotion, d'un spray ou d'un emplâtre transdermique.

11. Combinaison locale d'agents d'amplification de la pénétration dans la peau selon l'une quelconque des revendications précédentes 1 à 10, dans laquelle la combinaison de propylèneglycol et d'acide laurique dans un rapport 10:1 agit de manière synergique.

12. Utilisation d'une formulation pharmaceutique selon la revendication 1, pour la préparation d'une combinaison pharmaceutique locale destinée à empêcher ou à soulager une douleur aiguë d'incision et une neuropathie périphérique chronique chez un patient ayant besoin d'un tel traitement.

13. Utilisation d'une formulation pharmaceutique selon la revendication 1, pour la préparation d'une combinaison pharmaceutique locale destinée à empêcher ou à soulager une inflammation neuropathique chez un patient ayant besoin d'un tel traitement.

14. Utilisation selon la revendication 12 ou 13, dans laquelle l'administration de la composition pharmaceutique s'adresse à des surfaces cutanées, muqueuses, vaginales, rectales, oculaires ou nasales.
